# EUROPÄISCHE PATENTSCHRIFT

(11) **EP 0 875 494 B1**
(45) Veröffentlichungstag und Bekanntmachung des Hinweises auf die Patenterteilung: **31.10.2001**
(21) Anmeldenummer: 98107697.9
(22) Anmeldetag: 28.04.1998
(51) Int. Cl.: C07C 37/06

(54) **Verfahren zur Herstellung von 3,5-Dimethylphenol**
Process for the preparation of 3,5-dimethyl phenol
Procédé de préparation du 3,4-diméthyl phénol

(30) Priorität: 03.05.1997 DE 19718852
(43) Veröffentlichungstag der Anmeldung: 04.11.1998
(73) Patentinhaber: Rütgers VFT AG, 44579 Castrop-Rauxel (DE)
(72) Erfinder: Bergins, Wolfgang, 44579 Castrop-Rauxel (DE); Talbiersky, Jörg, Dr., 46282 Dorsten (DE)
(74) Vertreter: Cohausz & Florack

(56) Entgegenhaltungen:
- DE-C- 1 768 875

## Beschreibung

Die Erfindung betrifft ein Verfahren zur Herstellung von 3,5-Dimethylphenol durch katalytische Dimethylierung von Isophoron in der Gasphase in Gegenwart eines Metallkatalysators.

Ein entsprechendes Verfahren ist aus der DE 1 768 875 B1 bekannt, in dem bei einer Kontaktzeit von etwa 135 Sekunden Restisophorongehalte von 4 bis 17% im Rohprodukt festgestellt wurden. Die maximale Laufzeit des Laborreaktors betrug 135 Stunden. In der großtechnischen Übertragung des Verfahrens dieser Patentschrift unter Verwendung eines wälzgasbeheizten Rohrreaktors zeigte sich, daß hohe Laufzeiten des Reaktors von 1500 bis 2000 Stunden nur bei einer relativ niedrigen Kontaktzeit von 41 Sekunden zu erreichen sind. Um eine weitere Verkürzung der Laufzeit des Reaktors durch Kohlenstoffbildung auf dem Katalysator zu vermeiden, muß frischer Katalysator eingesetzt werden. Bei einer Einspeisung von 450 l/h, einer Reaktionstemperatur von 550°C und 10 bar Druck lag der Restisophorongehalt im Rohprodukt bei etwa 9%.

Wird anschließend das rohe 3,5-Dimethylphenol durch Kristallisation gereinigt, ist die hohe Isophoronkonzentration von erheblichem Nachteil; Isophoron geht in die Mutterlauge über, die zur Gewinnung des darin enthaltenen 3,5-Dimethylphenol rektifiziert wird. Da Isophoron und 3,5-Dimethylphenol ein Azeotrop bilden, wirkt Isophoron in der Destillation ausbeutemindernd oder macht eine Sonderbehandlung der 3,5-Dimethylphenol/Isophoron-Fraktion erforderlich. Nach Umlaugung und Destillation wird 3,5-Dimethylphenol mit einer Reinheit von 96% erhalten. Insgesamt schwankt die Ausbeute an 3,5-Dimethylphenol nach diesem Verfahren zwischen 55 und 60%, bezogen auf eingesetztes Isophoron.

Auch die Rückführung von mehrfach benutztem, regeneriertem Katalysator in den Reaktor bereitet Probleme, da sich beispielsweise durch die Oberflächenvergrößerung die Reaktivität des Katalysators deutlich erhöht und so das Verfahren schwer kontrollierbar wird.

Der Erfindung liegt somit die Aufgabe zugrunde, ein Verfahren bereitzustellen, das hohe Laufzeiten des Reaktors bei niedrigen Restisophorongehalten von kleiner 3%, eine damit verbundene gesteigerte Ausbeute an 3,5-Dimethylphenol und eine Einsparung an Katalysator ermöglicht.

Gelöst wird diese Aufgabe durch ein Verfahren zur Herstellung von 3,5-Dimethylphenol mittels katalytischer Dimethylierung von Isophoron in der Gasphase in Gegenwart eines Metalls oder einer Metallegierung als Katalysator, wobei man einen Isophoron enthaltenden Prozeßstrom durch Reaktionszonen führt, die den Katalysator in abgestufter Reaktivität enthalten, und der Prozeßstrom zwischen diesen Reaktionszonen thermostatisiert wird.

Unter abgestufter Reaktivität wird erfindungsgemäß verstanden, daß die Reaktionszonen im Reaktor Katalysator in unterschiedlicher Aktivität enthalten. Unterschiedliche Reaktivität des Katalysators kann dadurch erreicht werden, daß frischer oder gebrauchter Katalysator oder Gemische von beiden eingesetzt werden. Gemäß einer bevorzugten Ausführungsform wird der Prozeßstrom nacheinander über einen frischen, regenerierten und dann wieder über einen frischen Katalysator geleitet. Darüber hinaus können zwischen den beiden Zonen mit frischem Katalysator auch zwei Reaktionszonen eingerichtet werden, die regenerierten Katalysator enthalten und wiederum durch eine sogenannte Abkühlzone voneinander getrennt sind.

Zwischen den Katalysator enthaltenden Reaktionszonen erfährt der Prozeßstrom zur Abgabe von Reaktionswärme in den Abkühlzonen eine Thermostatisierung. Diese kann dadurch erfolgen, daß in der Abkühlzone keine Reaktion und damit keine zusätzliche Aufwärmung des bereits Endprodukt enthaltenden Prozeßstroms erfolgt.

Die Reaktion erfolgt exotherm. Als Reaktor wird vorzugsweise ein Rohrreaktor eingesetzt, der mit Wälzgas beheizt werden kann. Der Rohrreaktor ist derart mit Katalysator befüllt, daß sich mit Katalysator befüllte und von Katalysator freie Zonen abwechseln. Als Katalysatoren sind Metallkatalysatoren und Metallegierungskatalysatoren geeignet, beispielsweise Stahllegierungen. Bevorzugte Katalysatoren sind Chromnickelstahllegierungen, beispielsweise die Legierung X12 CrNi 18 8 (DIN 17006). Die Reaktionstemperatur des erfindungsgemäßen Verfahrens liegt vorzugsweise zwischen 450 und 600 °C.

Mit dem erfindungsgemäßen Verfahren werden Restisophorongehalte im Rohprodukt von nur 1,5% erhalten. Dies entspricht einer Ausbeuteverbesserung um 5% gegenüber dem bekannten Verfahren. Die Laufzeit beträgt dabei 2000 Stunden. Ferner kann ein mehrfach regenerierter Katalysator, dessen Reaktivität um den Faktor ≤ 10 gegenüber frischem Katalysator erhöht ist, ohne Verminderung der Laufzeit zu etwa 60% wiederverwendet werden. Dies führt zu einer deutlichen Kosteneinsparung beim Katalysatorverbrauch.

Das erfindungsgemäße Verfahren wird durch das folgende Beispiel näher erläutert.

### BEISPIEL

Eingesetzt wurde ein Rohrreaktor aus einer Chromnickelstahl-Legierung mit Kolonnenfüllkörpern aus dem gleichen Material. Der Reaktor war derart befüllt worden, daß in einer ersten Reaktionszone frischer Katalysator angeordnet war, auf den eine katalysatorfreie Zone im Rohrreaktor folgte. Daran schloß sich eine Reaktionszone mit regeneriertem Katalysator, eine katalysatorfreie Zone und daran anschließend wieder eine Reaktionszone mit regeneriertem Katalysator an. An diese Reaktionszone schloß sich unmittelbar eine weitere Reaktionszone mit frischem Katalysator an.

Die Aktivität des regenerierten Katalysators in den jeweiligen Zonen des Reaktors war im Vergleich zu dem frischen Katalysator im Reaktor um etwa den Faktor 4, entsprechend einer Laufzeit von etwa 1800 Stunden, erhöht. Das Volumen des Reaktors betrug 450 l.

Stündlich wurden bei 552°C und 9,5 bar 458 kg Isophoron durchgesetzt. Die Verweilzeit betrug 72 Sekunden, die Versuchsdauer 1600 Stunden. Die effektiven Volumina des frischen Kontaktes betrugen je 75 ml, die des regenerierten je 56 ml und die der katalysatorfreien Zonen je 94 ml.

Der Restisophorongehalt im Rohprodukt betrug 1,5 %. Die Ausbeute lag bei 63 % 3,5-Dimethylphenol, bezogen auf das Gewicht des eingesetzten Isophorons.

## Patentansprüche

1. Verfahren zur Herstellung von 3,5-Dimethylphenol durch katalytische Dimethylierung von Isophoron in der Gasphase in Gegenwart eines Katalysators aus einem Metall oder einer Metallegierung, **dadurch gekennzeichnet, daß** man einen Isophoron enthaltenden Prozeßstrom durch Reaktionszonen führt, die den Katalysator in abgestufter Reaktivität enthalten und den Prozeßstrom zwischen diesen Reaktionszonen thermostatisiert.

2. Verfahren nach Anspruch 1, **dadurch gekennzeichnet, daß** frischer Katalysator im Ein- und Ausgangsbereich des Reaktors und regenerierter reaktiver Katalysator im Mittelteil des Reaktors angeordnet sind.

## Claims

1. A process for the preparation of 3,5-dimethylphenol by catalytic dimethylation of isophorone in the gas phase in the presence of a catalyst consisting of a metal or a metal alloy, **characterized in that** an isophorone-containing process stream is guided through reaction zones containing said catalyst with different reactivity and the temperature of the process stream between said reaction zones is thermostated.

2. A process according to claim 1 **characterized in that** the fresh catalyst is located at the entrance and exit areas of the reactor, and regenerated reactive catalyst is applied to the middle section of the reactor.

## Revendications

1. Procédé de préparation de 3,5-diméthylphénol par diméthylation catalytique d'isophorone, en phase gazeuse et en présence d'un catalyseur en métal ou en un alliage métallique, **caractérisé en ce qu'**on conduit un courant contenant l'isophorone dans des zones de réaction, qui contiennent le catalyseur avec une réactivité décroissante, et que le courant est thermostatisé entre ces zones.

2. Procédé suivant la revendication 1, **caractérisé en ce que** le catalyseur frais est disposé dans les parties d'entrée et de sortie du réacteur, et le catalyseur régénéré plus réactif dans la partie centrale du réacteur.
